# EUROPEAN PATENT SPECIFICATION

(11) **EP 3 037 132 B1**
(45) Date of publication and mention of the grant of the patent: **31.10.2018**
(21) Application number: 15201485.8
(22) Date of filing: 21.12.2015
(51) Int. Cl.: A61P 25/28, A61K 9/00, A61K 31/315, A61K 31/353, A61K 33/30, A61K 9/08

(54) **ZINC SALT AND POLYPHENOL COMPOSITION**
ZINKSALZ UND POLYPHENOLZUSAMMENSETZUNG
SEL DE ZINC ET COMPOSITION DE POLYPHÉNOL

(30) Priority: 23.12.2014 IT GE20140133
(43) Date of publication of application: 29.06.2016
(73) Proprietor: Farma-Derma S.R.L., 40010 Sala Bolognese, BO (IT)
(72) Inventor: RUSSO, Vincenzo, 40137 Bologna, BO (IT)
(74) Representative: De Anna, Pier Luigi

(56) References cited:
- WO-A2-02/09699
- US-A1- 2006 039 954
- DARRELL SAWMILLER ET AL: "Luteolin Reduces Alzheimer's Disease Pathologies Induced by Traumatic Brain Injury", INTERNATIONAL JOURNAL OF MOLECULAR SCIENCES, vol. 15, no. 1, 1 January 2014 (2014-01-01), pages 895-904, XP055208646, DOI: 10.3390/ijms15010895
- MALUTA S MUFAMADI ET AL: "Surface-Engineered Nanoliposomes by Chelating Ligands for Modulating the Neurotoxicity Associated with [beta]-Amyloid Aggregates of Alzheimer's disease", PHARMACEUTICAL RESEARCH, KLUWER ACADEMIC PUBLISHERS-PLENUM PUBLISHERS, NL, vol. 29, no. 11, 15 May 2012 (2012-05-15), pages 3075-3089, XP035126577, ISSN: 1573-904X, DOI: 10.1007/S11095-012-0770-0

## Description

The present invention relates to a composition containing a zinc salt and a polyphenol. In particular the polyphenol is a particular hydroxyflavone and the zinc salt is a zinc salt of a carboxylic acid. The composition according to the invention is stable both as solid form and liquid solution. In particular the composition according to the invention is used as a therapeutic agent, such as for example in the treatment of Mild Cognitive Impairment (MCI) and Alzheimer's disease (AD). The preferred intake form is by oral route as capsules or tablets, or liquid solution.

Scientific studies demonstrated that an impaired homeostasis of metals could promote, through oxidative stress, the onset of Alzheimer's disease. Recent genetic and epidemiological studies confirmed such results showing that the excess of serum copper not bound to ceruloplasmin increases the risk of developing the disease.

### BACKGROUND OF THE PRIOR ART

Alzheimer's disease (AD) is the most common form of dementia, which affects 15 millions of people worldwide.

Depending on the age of onset, two forms are identified: an early-onset form, occurring before 65 years (1-5% of the cases), and a late-onset form, occurring after 65 years.

The early form is rare and has a Mendelian genetic autosomal dominant transmission. The late form represents approximately 95% of all the cases of Alzheimer's disease and its epidemiology is influenced by several risk factors, some of them being genetically transmissible, such as the epsilon4 form of the APOE gene (apolipoprotein E) and the familial inheritance, others comprising environmental factors, such as the age, traumatic brain injury, viral agents, hypercholesterolemia and metabolic disorder of metals, specifically copper.

AD is clinically characterized by a loss of memory and progressive cognitive decline. From a pathological point of view, the two elements which characterize the disease are intraneuronal neurofibrillary tangles containing the hyper-phosphorylated protein tau and extracellular amyloid plaques, formed by the insoluble beta-amyloid (Aβ) protein, deriving from the irregular cleavage of the amyloid precursor protein (APP).

Recent studies assume that metal ions, specifically copper (Cu²⁺), can be involved in the etiology of AD.

The copper metabolic disorder is already obvious in subjects with MCI and among them, those with high values of free copper (greater than 1.9 µM) have a 3-fold greater likelihood of developing dementia than subjects with MCI, but normal copper values.

Copper, as a catalyst of redox reactions, is an essential co-factor for many proteins and enzymes involved in the energy production, oxygen transport, hematopoiesis, cell growth, metabolism and signal transduction. Furthermore, copper is crucial for the maintenance of the physiological functioning of the cell and the development of the central nervous system.

Copper homeostasis is determined by the equilibrium between the copper absorbed from the diet and the excretion through feces and bile.

In healthy subjects, the presence of large amounts of copper in the diet poorly influences the absorption, in that metallothioneins, which are proteins present at the intestinal mucosa, regulate the levels of essential metals, among which those of copper. Metallothioneins entrap the copper and, following to the disintegration of the epithelium, are excreted with the feces along with the chelated copper. The expression of these proteins is modulated by the concentration of zinc ions.

Usually, 85-95% of serum copper is tightly bound to ceruloplasmin. The remainder, referred to as "free copper", is bound and exchanged among albumin, alpha-2-macroglobulin and low molecular weight compounds such as peptides and amino acids. The main difference between these two types of copper is that free copper is able to cross the blood-brain barrier.

A down-regulation of copper metabolism contributes to the pathogenic pathways of AD by forming reactive oxygen species (ROS). Particularly, in the AD, the free copper is able to enhance the APP dimerization, favoring the formation of Aβ aggregates.

They bind together to form plaques, which have a toxic effect and lead to oxidative stress as a consequence of ROS formation (R. Squitti. Copper subtype of Alzheimer's disease (AD): Meta-analyses, genetic studies and predictive value of non-ceruloplasmin copper in mildcognitive impairment conversion to full AD. Journal of Trace Elements in Medicine and Biology Volume 28, Issue 4, October 2014, Pages 482-485*;* R. Squitti, M. Siotto, R. Polimanti. Low-copper diet as a preventive strategy for Alzheimer's disease. Neurobiology of Aging 35 (2014) S40-S50).

Recent scientific evidences further demonstrated that another metal ion, the zinc, plays also a crucial role in the molecular mechanisms of memory and learning and that a diet poor in this element can cause disorders or injuries of the mnemonic functions.

The zinc enters the CNS as bound to amino acids, particularly L-histidine and cysteine and mainly concentrates in the hippocampus, amygdala and cerebellum, i.e. the areas involved in memory, learning and cognitive pathways.

Indeed, several experiments demonstrated that the zinc plays a crucial role in the molecular mechanisms of memory and learning, since it stimulates the receptors involved in these processes.

Moreover, as described above, the zinc regulates the production of metallothioneins, which represent the primary defence against the build-up of free copper. Zinc-deficient subjects produce less metallothioneins and thus they are more exposed to a high level of blood copper, which can then reach the Central Nervous System impairing the fragile mechanism of memory.

Contrary to copper, neither repository sites nor buffer proteins (in the case of copper, the ceruloplasmin) able to store zinc exist, and for this reason it should be taken daily (Malgorzata Tyszka-Czochara et al. The role of zinc in the pathogenesis and treatment of central nervous system (CNS) diseases. Implications of zinc homeostasis for proper CNS function. Acta Poloniae Pharmaceutica - Drug Research, vol. 71 no. 3 pp. 369-377, 2014).

The aim of the present invention is to develop a new combination of substances able to mitigate the adverse effects due to a zinc deficit and a free copper excess to the Central Nervous System, and counteract the oxidative damage resulting from the formation of beta-amyloid plaques.

The present project intends to test a formulation containing zinc and luteolin in order to:
✔ Prevent the risk related to an excess of non-ceruloplasmin copper
✔ Prevent the worsening of physical and mental disabilities

The present invention is directed to the use of compositions containing zinc-luteolin, which favor the elimination of the excess free copper and counteract the oxidative stress in patients with Mild Cognitive Impairment or Alzheimer's disease, in order to slow the progression of these diseases.

US 2006/0039954 A1 discloses a nutrient formulation for preventing or treating at least one ocular disorder in mammals. In this nutrient formulation, the zinc dosages are substantially reduced to an amount of about 15 to 40 mg elemental zinc, in order to reduce the risk of Alzheimer's disease and other neurotoxic damage in the brains of elderly people.

D. Sawmiller et al., Int. J. Mol. Sci. 2014, vol. 15, pages 895 to 94 discloses that luteolin reduces Alzheimer's disease pathologies induced by traumatic brain injury.

M. S. Mufamadi et al., Pharm Res. 2012, vol. 29, pages 3075 to 3089 discloses that Zinc acetate can restore biometal homeostasis and prevent or reverse undesired Aβ aggregation forming plaques which may result in Alzheimer's disease.

### SUMMARY OF THE INVENTION

The present invention concerns a new formulation as tablets or capsules, containing zinc and a polyphenol of formula (I) for use in the treatment of Mild Cognitive Impairment (MCI) and Alzheimer's disease (AD) according to claim 1 or a liquid composition according to claim 6. The scope of the present invention is defined by the appended claims. Any other disclosure in the present description, regardless of whether it is indicated as preferred or exemplified, does not form part of the present invention. In particular, the polyphenol of formula (I) 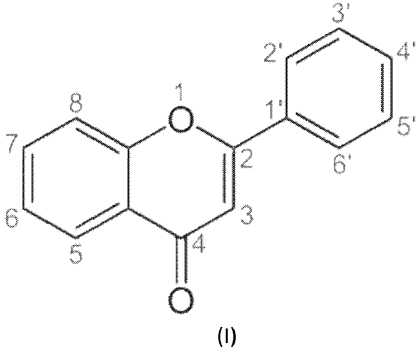 is selected from the group formed by 3',4',5,7-tetrahydroxyflavone, 4',5,7-trihydroxyflavone, 4',5,6,7,8-pentamethoxyflavone, 6-hydroxyflavone, 5,6,7-trihydroxyflavone, 5,6,7,4'-tetrahydroxyflavone; preferably the polyphenol is 3',4',5,7-tetrahydroxyflavone; whereas the zinc salt is a zinc salt of a carboxylic acid, preferably acetic acid.

The composition according to the present invention is used as a therapeutic agent in the treatment of Mild Cognitive Impairment (MCI) and Alzheimer's disease (AD).

In the forms for therapeutic use the composition according to the present invention is orally taken as capsules or tablets, or liquid solution. According to a preferred embodiment the composition is a solid mixture as a tablet or a capsule and the amount of zinc ion is within 20 and 60 mg for a tablet or a capsule, preferably the amount of zinc ion is within 25 and 35 mg for a tablet or a capsule. The polyphenol of formula (I) is preferably a hydroxyflavone and the amount of hydroxyflavone is within 10 and 50 mg for a tablet or a capsule. Preferably the hydroxyflavone is 3',4',5,7-tetrahydroxyflavone and the amount of 3',4',5,7-tetrahydroxyflavone is within 10 and 25 mg for a tablet or a capsule, more preferably within 10 and 22.5 mg for a tablet or a capsule.

According to another preferred embodiment the composition is a liquid solution and the amount of zinc ion is within 2 and 20 mg for 1 ml of solution, preferably the amount of zinc ion is within 4 and 8 mg for 1 ml of solution, whereas the amount of hydroxyflavone, preferably 3',4',5,7-tetrahydroxyflavone, is within 1 and 20 mg for 1 ml of solution. The concentration of 3',4',5,7-tetrahydroxyflavone is preferably within 2 and 6 mg for 1 ml of solution, more preferably within 3.5 and 4.5 mg for 1 ml of solution.

### DESCRIPTION OF THE EMBODIMENTS OF THE INVENTION

According to clinical studies developed by the inventors, the zinc, being used in the formulation as salt form, was determined to compensate the deficit of this element in subjects with MCI or at risk of Alzheimer's disease, and induce the duodenal production of metallothioneins, which are low molecular weight proteins (from 500 to 14.000 Da), located in the Golgi apparatus, characterized by a rich content of cysteine and a high affinity to heavy metals. These proteins are involved in the regulation of essential metals (zinc and copper) and play a protective role against the heavy metal toxicity as well as the oxidative stress. The production thereof relies on the availability of essential elements, such as zinc, copper and selenium, as well as the availability of the amino acids histidine and cysteine.

Flavones are a subclass of naturally occurring chemicals belonging to the class of flavonoids. Natural flavones include luteolin (3',4',5,7-tetrahydroxyflavone), apigenin (4',5,7-trihydroxyflavone), tangeritin (4',5,6,7,8-pentamethoxyflavone), 6-hydroxyflavone, baicalein (5,6,7-trihydroxyflavone), 5,6,7,4'-tetrahydroxyflavone.

Recent studies shown that this class of compounds, due to their antioxidant ability, is able to counteract ROS as well as the oxidative stress involved in the neuronal death induced by beta-amyloid plaques (Fu X, Zhang J et al. Protective role of luteolin against cognitive dysfunction induced by chronic cerebral hypoperfusion in rats. Pharmacol Biochem Behav. 2014 Nov;126:122-30)*.*

MCI condition is now considered a predictive factor for the onset of Alzheimer's disease. The copper metabolic disorder is already obvious in subjects with MCI and among them, those with a high blood concentration of free copper have a 3-fold greater likelihood of developing dementia than subjects affected by MCI with normal levels of the element. The fraction of copper, which results to be involved in the processes of the disease onset, is that not bound to the protein ceruloplasmin, which is able to cross the blood-brain barrier accelerating the beta-amyloid cascade, which is considered the cause of brain neurodegeneration.

The combination of polyphenolic flavones of formula (I) with zinc ions as salt form, of carboxylic acids, administered to patients with MCI or early-stage Alzheimer's disease, by enhancing the duodenal production of metallothioneins, regulates the build-up of other metals such as copper. The synergistic effect of polyphenolic flavones counteracts both ROS and oxidative stress involved in the neuronal death induced by beta-amyloid plaques.

According to a preferred embodiment the composition is a solid mixture of zinc salts/polyphenol as a tablet or a capsule and the amount of zinc ion is within 20 and 60 mg for a tablet or a capsule, preferably the amount of zinc ion is within 25 and 35 mg for a tablet or a capsule; the polyphenol is a hydroxyflavone and the amount of hydroxyflavone is within 10 and 50 mg for a tablet or a capsule. The hydroxyflavone can be 3',4',5,7-tetrahydroxyflavone, preferably the amount of 3',4',5,7-tetrahydroxyflavone is within 10 and 25 mg for a tablet or a capsule, more preferably within 10 and 22.5 mg for a tablet or a capsule.

According to another preferred embodiment the composition is a liquid composition and the amount of zinc ion is within 2 and 20 mg for 1 ml of solution, preferably the amount of zinc ion is within 4 and 8 mg for 1 ml of solution. Again, according to this embodiment the hydroxyflavone could be 3',4',5,7-tetrahydroxyflavone and the amount of 3',4',5,7-tetrahydroxyflavone is within 1 and 20 mg for 1 ml of solution, preferably 3',4',5,7-tetrahydroxyflavone is within 2 and 6 mg for 1 ml of solution, more preferably within 3.5 and 4.5 mg for 1 ml of solution. The solution is both an aqueous and an aqueous alcoholic solution.

A particularly preferred embodiment of the formulation as tablets or capsules comprises the following amounts of substances for a capsule or a tablet:
25 mg of zinc ion as acetate and 10 mg of 3',4',5,7-tetrahydroxyflavone.

A particularly preferred embodiment of the formulation as liquid solution comprises the following amounts of substances for 1 ml:
6.25 mg of zinc ion as acetate and 4 mg of 3',4',5,7-tetrahydroxyflavone.

### EXAMPLES

A group of 60 patients was divided into three groups:
1) 20 Subjects with MCI having a copper value within 1.6 µM and 2.3 µM;
2) 20 Subjects with overt Alzheimer's disease having a copper value > 2.3 µM;
3) 20 Placebo Subjects.

The 60 subjects were followed for 6 months: the first monitoring was performed at the beginning of the treatment, and the following ones at the third and sixth months. The three groups were analyzed by the following studies:
- Measurement of serum copper;
- Measurement of urine zinc (zincuria);
- Cognitive tests such as Mini Mental Examination;

Primary aims of the test:
- Assessing the safety and tolerability of the supplement product based on Zinc-Luteolin as compared to placebo for 6 months;
- Determining the efficacy of the treatment

**Tab. 1 - Assessment of the safety, tolerability and efficacy of the supplement product based on Zinc-Luteolin - 750 mg tablets containing 22.5 mg luteolin and 30 mg zinc ion.**

| 2 times/die tablets 750mg | **Initial** | | | **three months** | | | **six months** | | |
|---|---|---|---|---|---|---|---|---|---|
| ***solid formulat.22.5 mg luteolin and 30 mg zinc ion.*** | *Serum copper (µM)* | Zincuria (µM) | *MME* | *Serum copper (µM)* | Zincuria (µM) | *MME* | *Serum copper (µM)* | Zincuria (µM) | *MME* |
| Subjects with MCI | *2,3* | 364 | 20 | *2* | 370 | *21* | *2* | 372 | *21* |
| | *2,1* | 241 | 21 | *1,9* | 245 | *20* | *1,7* | 252 | *21* |
| | *1,75* | 408 | 19 | *1,5* | 411 | *19* | *1,5* | 413 | *21* |
| | *1,9* | 311 | 23 | *1,6* | 312 | *23* | *1,7* | 319 | *23* |
| | *2* | 269 | 21 | *1,8* | 273 | *22* | *1,7* | 277 | *22* |
| Subjects with overt Alzheimer's disease | *2,8* | 312 | 15 | *2,5* | 314 | *15* | *2,2* | 316 | *16* |
| | *2,4* | 245 | 12 | *2* | 248 | *12* | *2* | 252 | *12* |
| | *2,5* | 406 | 16 | *2,1* | 410 | *18* | *1,9* | 413 | *18* |
| | *2,9* | 511 | 17 | *2,5* | 513 | *18* | *2,3* | 521 | *18* |
| | *2,5* | 294 | 16 | *2,3* | 297 | *16* | *2* | 301 | *17* |
| Placebo Subjects | *1,9* | 257 | 22 | *2* | 255 | *21* | *2,3* | 262 | *20* |
| | *2,3* | 368 | 21 | *2,3* | 369 | *19* | *2,5* | 352 | *18* |
| | *1,75* | 289 | 18 | *2* | 285 | *18* | *2* | 286 | *16* |
| | *2,2* | 220 | 20 | *2,5* | 217 | *19* | *2,7* | 221 | *19* |
| | *2,6* | 245 | 19 | *2,9* | 240 | *17* | *3,1* | 237 | *17* |

**Tab. 2 - Assessment of the safety, tolerability and efficacy of the supplement product based on Zinc-Luteolin - 500 mg tablets containing 10 mg luteolin and 25 mg zinc ion.**

| 4 times/die tablets 500mg | **Initial** | | | **three months** | | | **six months** | | |
|---|---|---|---|---|---|---|---|---|---|
| ***solid formulat. 10mg luteolin and 25 mg zinc ion.*** | *Serum copper (µM)* | Zincuria | *MME* | *Serum copper (µM)* | Zincuria | *MME* | *Serum copper (µM)* | Zincuria | *MME* |
| Subjects with MCI | *1,7* | 462 | *18* | *1,1* | 465 | *20* | *1,1* | 473 | *21* |
| | *1,7* | 325 | *21* | *1,5* | 331 | *21* | *1,1* | 341 | *22* |
| | *2,2* | 236 | *19* | *2* | 245 | *19* | *1,5* | 253 | *21* |
| | *2* | 258 | *23* | *1,5* | 264 | *23* | *1,3* | 278 | *24* |
| | *1,9* | 370 | *20* | *1,4* | 381 | *21* | *1* | 388 | *22* |
| Subjects with overt Alzheimer s disease | *2,7* | 269 | *15* | *2,2* | 275 | *16* | *1,9* | 282 | *17* |
| | *2,5* | 236 | *14* | *2* | 251 | *14* | *1,5* | 257 | *16* |
| | *2,6* | 345 | *17* | *2* | 361 | *18* | *1,8* | 363 | *19* |
| | *2,4* | 301 | *15* | *1,9* | 315 | *15* | *1,7* | 321 | *16* |
| | *2,5* | 462 | *16* | *2* | 472 | *17* | *1,7* | 477 | *19* |
| Placebo Subjects | *1,9* | 257 | *22* | *2* | 255 | *21* | *2,3* | 262 | *20* |
| | *2,3* | 368 | *21* | *2,3* | 369 | *19* | *2,5* | 352 | *18* |
| | *1,75* | 289 | *18* | *2* | 285 | *18* | *2* | 286 | *16* |
| | *2,2* | 220 | *20* | *2,5* | 217 | *19* | *2,7* | 221 | *19* |
| | *2,6* | 245 | *19* | *2,9* | 240 | *17* | *3,1* | 237 | *17* |

Results demonstrate that, for the placebo subjects, the administration of the formulation as tablets, tab. 1 and 2, according to the invention at the given dosages and frequencies do not change the values of the measurements of serum copper and urine zinc (zincuria); the cognitive tests Mini Mental Examination did not significantly change. The same administrations in subjects with MCI and Alzheimer's disease suggest that the values of the serum copper measurement are markedly reduced and that, despite of the zinc intake, the zinc amount in the urine (zincuria) remained basically unchanged; cognitive tests Mini Mental Examination in subjects with MCI and Alzheimer's disease significantly changed.

**Tab. 3 - Assessment of the safety, tolerability and efficacy of the supplement product based on Zinc-Luteolin - Liquid formulation containing 15 mg luteolin and 18 mg zinc ion per dose of 3 ml.**

| 2 times/die 3 ml | **Initial** | | | **three months** | | | **six months** | | |
|---|---|---|---|---|---|---|---|---|---|
| ***Liquid formulat.15 mg luteolin and 18 mg zinc ion.*** | *Serum copper (µM)* | Zincuria (µM) | *MME* | *Serum copper (µM)* | Zincuria (µM) | *MME* | *Serum copper (µM)* | Zincuria (µM) | *MME* |
| Subjects with MCI | *1,75* | 300 | *22* | *1,6* | 325 | *22* | *1,6* | 327 | *22* |
| | *2* | 234 | *24* | *1,75* | 236 | *25* | *1,8* | 250 | *25* |
| | *1,9* | 451 | *21* | *1,7* | 462 | *21* | *1,7* | 460 | *22* |
| | *1,9* | 327 | *21* | *1,8* | 335 | *20* | *1,7* | 341 | *21* |
| | *2,2* | 248 | *23* | *2* | 251 | *24* | *2* | 255 | *24* |
| Subjects with overt Alzheimer's disease | *2,5* | 509 | *17* | *2,4* | 523 | *17* | *2,2* | 522 | *18* |
| | *2,4* | 261 | *18* | *2,3* | 270 | *18* | *2,3* | 273 | *18* |
| | *2,4* | 279 | *18* | *2,2* | 283 | *19* | *2,2* | 289 | *18* |
| | *2,7* | 456 | *16* | *2,7* | 459 | *17* | *2,5* | 463 | *16* |
| | *2,9* | 387 | *15* | *2,6* | 394 | *16* | *2,8* | 396 | *16* |
| Placebo Subjects | *1,7* | 361 | *23* | *1,8* | 360 | *22* | *1,9* | 365 | *22* |
| | *2,3* | 285 | *18* | *2,5* | 287 | *17* | *2,7* | 284 | *15* |
| | *1,9* | 472 | *20* | *2* | 465 | *19* | *2* | 466 | *18* |
| | *2,5* | 301 | *19* | *2,6* | 314 | *19* | *2,8* | 303 | *19* |
| | *2,6* | 232 | *22* | *2,8* | 230 | *20* | *3* | 231 | *19* |

**Tab. 4 - Assessment of the safety, tolerability and efficacy of the supplement product based on Zinc-Luteolin - Liquid formulation containing 24 mg luteolin and 45 mg zinc ion per dose of 3 ml.**

| 2 times/day 3 ml | **Initial** | | | **three months** | | | **six months** | | |
|---|---|---|---|---|---|---|---|---|---|
| ***Liquid formulat. 24 mg luteolin and 45 mg zinc ion.*** | *Serum copper (µM)* | Zincuria (µM) | *MME* | *Serum copper (µM)* | Zincuria (µM) | *MME* | *Serum copper (µM)* | Zincuria (µM) | *MME* |
| Subjects with MCI | *1,7* | 559 | *20* | *1,3* | 570 | *21* | *1* | 573 | *22* |
| | *2,3* | 413 | *21* | *2* | 426 | *20* | *1,7* | 430 | *21* |
| | *2,2* | 289 | *22* | *1,8* | 296 | *24* | *1,7* | 305 | *24* |
| | *1,9* | 320 | *23* | *1,3* | 337 | *23* | *1,5* | 339 | *24* |
| | *1,7* | 240 | *21* | *1,2* | 251 | *21* | *1* | 254 | *22* |
| Subjects with overt Alzheimer's disease | *2,5* | 213 | *17* | *2* | 220 | *18* | *1,9* | 231 | *18* |
| | *2,9* | 305 | *18* | *2,5* | 312 | *18* | *2,1* | 320 | *20* |
| | *2,4* | 256 | *18* | *2* | 271 | *19* | *1,9* | 275 | *20* |
| | *2,6* | 453 | *16* | *2,1* | 468 | *16* | *2,2* | 475 | *18* |
| | *2,6* | 400 | *16* | *2,3* | 409 | *17* | *2,2* | 415 | *17* |
| Placebo Subjects | *1,7* | 361 | *23* | *1,8* | 360 | *22* | *1,9* | 365 | *22* |
| | *2,3* | 285 | *18* | *2,5* | 287 | *17* | *2,7* | 284 | *15* |
| | *1,9* | 472 | *20* | *2* | 465 | *19* | *2* | 466 | *18* |
| | *2,5* | 301 | *19* | *2,6* | 314 | *19* | *2,8* | 303 | *19* |
| | *2,6* | 232 | *22* | *2,8* | 230 | *20* | *3* | 231 | *19* |

Similarly, the results demonstrate that, for placebo subjects, the administration of the formulation as liquid form according to the invention at the given dosages and frequencies do not change the values of the measurements of serum copper and urine zinc (zincuria); cognitive tests Mini Mental Examination did not significantly change. The same administrations in subjects with MCI and Alzheimer's disease suggest that the values of the serum copper measurement are markedly reduced and that, despite of the zinc intake, the zinc amount in the urine (zincuria) remained basically unchanged; cognitive tests Mini Mental Examination in subjects with MCI and Alzheimer's disease significantly changed.

These initial experimental results confirm that the oral administration of a formulation containing zinc and hydroxyflavone as capsules or tablets, or liquid solution promotes the elimination of the excess free copper. The administration of zinc as salt form compensates for the deficit of this element in subjects with MCI or Alzheimer's disease, and induces the duodenal production of metallothioneins thereby favoring the elimination of the excess free copper.

The combined action of a hydroxyflavone, such as 3',4',5,7-tetrahydroxyflavone, which, due to its antioxidant ability, is able to counteract ROS as well as oxidative stress involved in neuronal death induced by beta-amyloid plaques, leads to a surprising synergistic effect.

It should be noted, by the skilled in the field, that the present invention allows embodiments in many other specific forms without departing from the field of the invention as claimed. Therefore, the present embodiments should be considered as illustrative, but they can be modified within the field defined by the scope of the appended claims, and the invention should not be limited to the above details.

## Claims

1. A solid composition as a tablet or a capsule comprising:
a. a zinc salt of a carboxylic acid wherein the amount of zinc ion is within 20 and 60 mg for tablet or capsule, and
b. a polyphenol of formula (I) 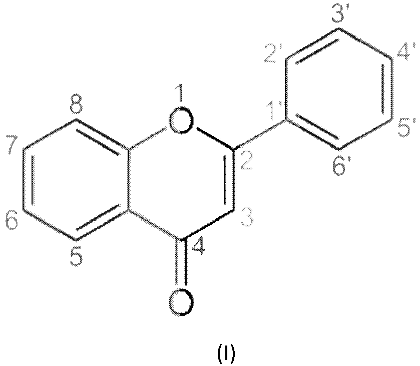 wherein the polyphenol (I) is selected from the group formed by 3',4',5,7-tetrahydroxyflavone, 4',5,7-trihydroxyflavone, 4',5,6,7,8-pentamethoxyflavone, 6-hydroxyflavone, 5,6,7-trihydroxyflavone and 5,6,7,4'-tetrahydroxyflavone the amount within 10 and 50 mg for each tablet or capsule; for use in the treatment of Mild Cognitive Impairment (MCI) and Alzheimer's disease (AD).

2. The solid composition for use in the treatment of MCI and AD according to claim 1, wherein carboxylic acid of zinc salt is acetic acid and the polyphenol is 3',4',5,7-tetrahydroxyflavone.

3. The solid composition for use in the treatment of MCI and AD according to claim 2, wherein the amount of zinc ion is within 25 and 35 mg for a tablet or a capsule.

4. The solid composition for use in the treatment of MCI and AD according to claim 3, wherein the amount of 3',4',5,7- tetrahydroxyflavone is within 10 and 25 mg for a tablet or a capsule.

5. The solid composition for use in the treatment of MCI and AD according to claim 4, wherein the amount of 3',4',5,7- tetrahydroxyflavone is within 10 and 22.5 mg for a tablet or a capsule.

6. A liquid composition comprising:
a. a zinc salt of a carboxylic acid wherein the amount of zinc ion is within 2 and 20 mg for 1 ml of solution, and
b. a polyphenol of formula (I) 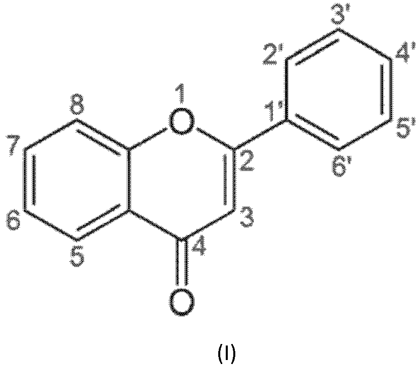 wherein the polyphenol (I) is selected from the group formed by 3',4',5,7-tetrahydroxyflavone, 4',5,7-trihydroxyflavone, 4',5,6,7,8-pentamethoxyflavone, 6-hydroxyflavone, 5,6,7-trihydroxyflavone and 5,6,7,4'-tetrahydroxyflavone; for use in the treatment of Mild Cognitive Impairment (MCI) and Alzheimer's disease (AD).

7. The liquid composition for use in the treatment of MCI and AD according to claim 6, wherein the carboxylic acid of zinc salt is acetic acid and the polyphenol is 3',4',5,7-tetrahydroxyflavone.

8. The liquid composition for use in the treatment of MCI and AD according to claim 7, wherein the amount of zinc ion is within 4 and 8 mg for 1 ml of solution.

9. The liquid composition for use in the treatment of MCI and AD according to claim 8, wherein the amount of 3',4',5,7- tetrahydroxyflavone is within 1 and 20 mg for 1 ml of solution.

10. The liquid composition for use in the treatment of MCI and AD according to claim 9, wherein the amount of 3',4',5,7- tetrahydroxyflavone is within 2 and 6 mg for 1 ml of solution.

11. The liquid composition for use in the treatment of MCI and AD according to claim 10, wherein the amount of 3',4',5,7- tetrahydroxyflavone is within 3.5 and 4.5 mg for 1 ml of solution.

## Patentansprüche

1. Feste Zusammensetzung als eine Tablette oder eine Kapsel umfassend:
a) ein Zinksalz von einer Carbonsäure, wobei die Menge an Zinkion zwischen 20 und 60 mg für Tablette oder Kapsel beträgt, und
b) ein Polyphenol mit Formel (I) 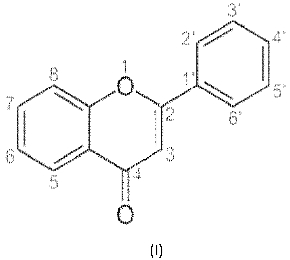 wobei das Polyphenol (I) aus der Gruppe ausgewählt ist, die durch 3',4',5,7-Tetrahydroxyflavon, 4',5,7-Trihydroxyflavon, 4',5,6,7,8-Pentamethoxyflavon, 6-Hydroxyflavon, 5,6,7-Trihydroxyflavon und 5,6,7,4'-Tetrahydroxyflavon gebildet wird, wobei die Menge zwischen 10 und 50 mg für jede Tablette oder Kapsel beträgt; zur Verwendung in der Behandlung von leichter kognitiver Beeinträchtigung (LKB) und Alzheimer-Krankheit (AK).

2. Feste Zusammensetzung zur Verwendung in der Behandlung von LKB und AK nach Anspruch 1, wobei die Carbonsäure vom Zinksalz Essigsäure ist und das Polyphenol 3',4',5,7-Tetrahydroxyflavon ist.

3. Feste Zusammensetzung zur Verwendung in der Behandlung von LKB und AK nach Anspruch 2, wobei die Menge an Zinkion zwischen 25 und 35 mg für eine Tablette oder eine Kapsel beträgt.

4. Feste Zusammensetzung zur Verwendung in der Behandlung von LKB und AK nach Anspruch 3, wobei die Menge an 3',4',5,7-Tetrhydroxyflavon zwischen 10 und 25 mg für eine Tablette oder eine Kapsel beträgt.

5. Feste Zusammensetzung zur Verwendung in der Behandlung von LKB und AK nach Anspruch 4, wobei die Menge an 3',4',5,7-Tetrahydroxyflavon zwischen 10 und 22,5 mg für eine Tablette oder eine Kapsel beträgt.

6. Flüssige Zusammensetzung umfassend:
a. ein Zinksalz von einer Carbonsäure, wobei die Menge an Zinkion zwischen 2 und 20 mg für 1 ml einer Lösung beträgt, und
b. ein Polyphenol mit Formel (I) 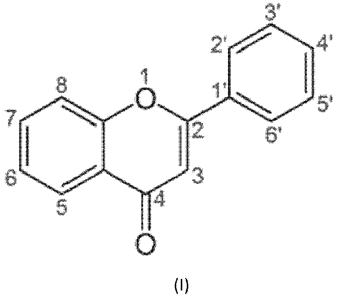 wobei das Polyphenol (I) aus der Gruppe ausgewählt ist, die durch 3',4',5,7-Tetrahydroxyflavon, 4',5,7-Trihydroxyflavon, 4',5,6,7,8-Pentamethoxyflavon, 6-Hydroxyflavon, 5,6,7-Trihydroxyflavon und 5,6,7,4'-Tetrahydroxyflavon gebildet wird; zur Verwendung in der Behandlung von leichter kognitiver Beeinträchtigung (LKB) und Alzheimer-Krankheit (AK).

7. Flüssige Zusammensetzung zur Verwendung in der Behandlung von LKB und AK nach Anspruch 6, wobei die Carbonsäure vom Zinksalz Essigsäure ist und das Polyphenol 3',4',5,7-Tetrahydroxyflavon ist.

8. Flüssige Zusammensetzung zur Verwendung in der Behandlung von LKB und AK nach Anspruch 7, wobei die Menge an Zinkion zwischen 4 und 8 mg für 1 ml Lösung beträgt.

9. Flüssige Zusammensetzung zur Verwendung in der Behandlung von LKB und AK nach Anspruch 8, wobei die Menge an 3',4',5,7-Tetrahydroxyflavon zwischen 1 und 20 mg für 1 ml Lösung beträgt.

10. Flüssige Zusammensetzung zur Verwendung in der Behandlung von LKB und AK nach Anspruch 9, wobei die Menge an 3',4',5,7-Tetrahydroxyflavon zwischen 2 und 6 mg für 1 ml Lösung beträgt.

11. Flüssige Zusammensetzung zur Verwendung in der Behandlung von LKB und AK nach Anspruch 10, wobei die Menge an 3',4',5,7-Tetrahydroxyflavon zwischen 3,5 und 4,5 mg für 1 ml Lösung beträgt.

## Revendications

1. Composition solide sous forme d'un comprimé ou d'une capsule comprenant :
a. un sel de zinc d'un acide carboxylique dans laquelle la quantité d'ion de zinc est comprise entre 20 et 60 mg pour un comprimé ou une capsule, et
b. un polyphénol de formule (I) 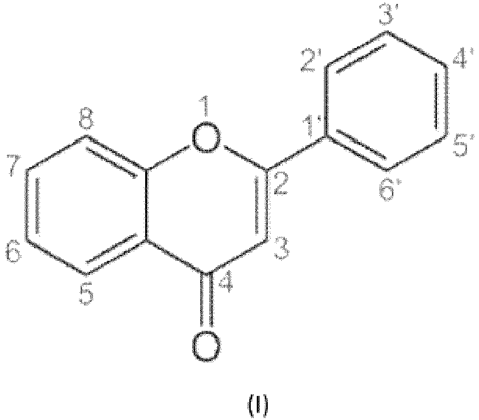 dans laquelle le polyphénol (I) est sélectionné dans le groupe formé par la 3',4',5,7-tétra-hydroxyflavone, la 4',5,7-trihydroxyflavone, la 4',5,6,7,8-pentaméthoxyflavone, la 6-hydroxyflavone, la 5,6,7-trihydroxyflavone et la 5,6,7,4'-tétrahydroxyflavone dont la quantité est comprise entre 10 et 50 mg pour chaque comprimé ou capsule ; pour son utilisation dans le traitement d'un trouble cognitif léger (TCL) et de la maladie d'Alzheimer (MA).

2. Composition solide pour son utilisation dans le traitement d'un TCL et de la MA selon la revendication 1, dans laquelle l'acide carboxylique du sel de zinc est l'acide acétique et le polyphénol est la 3',4',5,7-tétrahydroxyflavone.

3. Composition solide pour son utilisation dans le traitement d'un TCL et de la MA selon la revendication 2, dans laquelle la quantité d'ion de zinc est comprise entre 25 et 35 mg pour un comprimé ou une capsule.

4. Composition solide pour son utilisation dans le traitement d'un TCL et de la MA selon la revendication 3, dans laquelle la quantité de 3',4',5,7-tétrahydroxyflavone est comprise entre 10 et 25 mg pour un comprimé ou une capsule.

5. Composition solide pour son utilisation dans le traitement d'un TCL et de la MA selon la revendication 4, dans laquelle la quantité de 3',4',5,7-tétrahydroxyflavone est comprise entre 10 et 22,5 mg pour un comprimé ou une capsule.

6. Composition liquide comprenant :
a. un sel de zinc d'un acide carboxylique dans laquelle la quantité d'ion de zinc est comprise entre 2 et 20 mg pour 1 ml de solution, et
b. un polyphénol de formule (I) 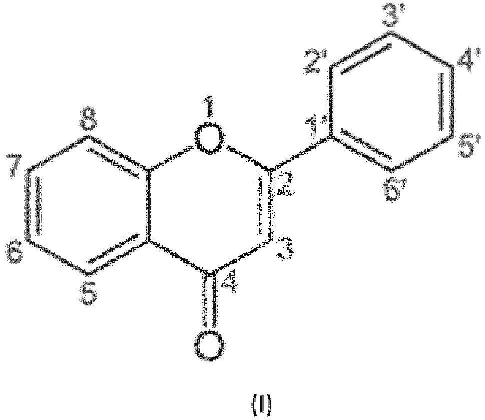 dans laquelle le polyphénol (I) est sélectionné dans le groupe formé par la 3',4',5,7-tétra-hydroxyflavone, la 4',5,7-trihydroxyflavone, la 4',5,6,7,8-pentaméthoxyflavone, la 6-hydroxyflavone, la 5,6,7-trihydroxyflavone et la 5,6,7,4'-tétrahydroxyflavone ; pour son utilisation dans le traitement d'un trouble cognitif léger (TCL) et de la maladie d'Alzheimer (MA).

7. Composition liquide pour son utilisation dans le traitement d'un TCL et de la MA selon la revendication 6, dans laquelle l'acide carboxylique du sel de zinc est l'acide acétique et le polyphénol est la 3',4',5,7-tétrahydroxyflavone.

8. Composition liquide pour son utilisation dans le traitement d'un TCL et de la MA selon la revendication 7, dans laquelle la quantité d'ion de zinc est comprise entre 4 et 8 mg pour 1 ml de solution.

9. Composition liquide pour son utilisation dans le traitement d'un TCL et de la MA selon la revendication 8, dans laquelle la quantité de 3',4',5,7-tétrahydroxyflavone est comprise entre 1 et 20 mg pour 1 ml de solution.

10. Composition liquide pour son utilisation dans le traitement d'un TCL et de la MA selon la revendication 9, dans laquelle la quantité de 3',4',5,7-tétrahydroxyflavone est comprise entre 2 et 6 mg pour 1 ml de solution.

11. Composition liquide pour son utilisation dans le traitement d'un TCL et de la MA selon la revendication 10, dans laquelle la quantité de 3',4',5,7-tétrahydroxyflavone est comprise entre 3,5 et 4,5 mg pour 1 ml de solution.
